# EUROPEAN PATENT SPECIFICATION

(11) **EP 3 533 061 B1**
(45) Date of publication and mention of the grant of the patent: **20.08.2025**
(21) Application number: 17790746.6
(22) Date of filing: 23.10.2017
(51) Int. Cl.: G16H 10/60, G16H 15/00

(54) **DEVICE, SYSTEM, AND METHOD FOR OPTIMIZING USAGE OF PRIOR STUDIES**
VORRICHTUNG, SYSTEM UND VERFAHREN ZUR OPTIMALEN NUTZUNG VON FRÜHEREN STUDIEN
DISPOSITIF, SYSTÈME ET PROCÉDÉ D'OPTIMISATION L'UTILISATION D'ÉTUDES ANTÉRIEURES

(30) Priority: 25.10.2016 US 201662412349 P
(43) Date of publication of application: 04.09.2019
(73) Proprietor: Koninklijke Philips N.V., 5656 AG Eindhoven (NL)
(72) Inventor: HOMBAL, Vadiraj, 5656 AE Eindhoven (NL); MANKOVICH, Gabriel, Ryan, 5656 AE Eindhoven (NL); TAHMASEBI MARAGHOOSH, Amir, Mohammad, 5656 AE Eindhoven (NL); QIAN, Yuechen, 5656 AE Eindhoven (NL)
(74) Representative: Philips Intellectual Property & Standards
(86) International application number: PCT/EP2017/076949
(87) International publication number: WO 2018/077776

(56) References cited:
- US-A1- 2014 288 970
- US-A1- 2016 147 946

## Description

### Background Information

A medical professional may perform a procedure on a patient and prepare a report of the results of the procedure. For example, a radiologist may perform a radiological study on the patient. To properly prepare the report, the radiologist may require a context of an imaging study so that the report includes the findings of the current study. However, the radiologist may also require a broader context to arrive at the context for the current study. For example, those skilled in the art will appreciate the need to know contexts such as disease progression, disease etiology, and/or general patient disease and health history to establish a baseline for the current study and arrive at useful conclusions. Thus, prior studies may provide invaluable information to the medical professional to prepare the report as well as for a variety of other reasons.

As those skilled in the art will understand, prior studies may provide information that may be used for a variety of reasons by radiologists. In a first example, the prior studies for a particular patient may provide a broader context to arrive at findings to be included in a report for a current study or current procedure performed on the patient. In a second example, a current imaging study or image may have insufficient information for the medical professional to properly investigate the issue of the patient. For example, there may be a poor image resolution or an inappropriate field-of-view to capture the target anatomy or finding under investigation. Thus, to extract a context, a radiologist refers to all the prior studies available for the patient that are relevant to the concerns regarding the current study. In either example, the prior studies must be determined for relevancy to be of importance to the radiologist. The prior studies perused by the radiologist to write a report on the current study are referred to as Relevant Prior Studies (RPS).

However, identifying prior studies of the patient as well as their relevancy to the current study is a laborious process that creates a major bottleneck in the radiology workflow. Specifically, the medical professional must manually search through the prior studies (sometimes only to discover the irrelevancy to the current study) or use an automated search which uses semantic parameters that only generates results that have an identical modality and body part to the current study.

The process of identifying the prior studies that are relevant depends on a variety of factors. In an exemplary factor, the prior studies that are relevant may depend on the type of analysis in which the radiologist is interested. For example, in observing disease progression, the radiologist may select a sequence of recent studies with a specific anatomy, affliction, and modality. On the other hand, in identifying a disease etiology, studies that are complementary to (and not identical to) the current modality or anatomy may be useful (e.g., a previous ultrasound of the lower abdomen may be complementary to a computed tomography (CT) scan or a functional study such as a positron emission tomography (PET) scan).

Due to the various factors relied upon in identifying the prior studies that are relevant, a manual approach in which the radiologist goes over all the prior studies associated with a patient is a major bottleneck in the radiology workflow that requires a significant amount of time if the search is to be exhaustive and complete. In practice, it is not realistic or reasonable for a radiologist to review all prior studies for a patient. Instead, the radiologist approximates the most relevant prior studies by their own criteria which in turn creates problems. In a first problem, the radiologist must look at a list of all prior studies of the patient and select one prior study that seems the most relevant. Such a process is inefficient in terms of time, and also prone to errors. Oftentimes, the radiologist opens a prior study and begins to review the selected prior study only to realize that the selected prior study is irrelevant. In a second problem, the criteria for selecting a prior study is variable across each individual radiologist, across different workflows, and dependent on the history of the patient. Accordingly, the process of identifying a prior study that is relevant is very challenging, particularly to implement the process in an automated manner.

In another operation related to relevant prior studies, during a typical radiology reading session, a radiologist may dictate findings and observations for a current study while looking at a radiology image (e.g., captured by the procedure device 105). The radiologist may also browse through prior imaging studies to find a similar/relevant case. For example, the current imaging study may not provide sufficient resolution for visualization of the targeted finding, the current imaging study does not cover the anatomy/finding of interest (e.g., incidental findings), and/or the radiologist is interested in comparing the observation in the current imaging study with prior relevant imaging studies to gather comparative and/or complementary information. In a specific example, the radiologist may be reading a chest CT image but may also want to look at prior chest CT images (if available) to evaluate a growth of a nodule in a lung or may want to look at a PET image to evaluate the functional response to a treatment. If the radiologist is not convinced that the current imaging study or the prior imaging studies serve the required purpose, the radiologist may recommend a new imaging study. That is, the radiologist may recommend the patient to undergo another procedure to capture further images that may assist in the diagnosis. The radiologist may also recommend a new imaging study for other reasons such as to conduct a follow-up study (e.g., for incidental findings). This recommendation may be included in a report for the current study.

Those skilled in the art will appreciate the frequency with which a radiologist will recommend that another imaging study be ordered for the patient. However, the recommended imaging study may be unnecessary as a similar imaging study may have been acquired from the patient in the past that would disregard the recommendation. Therefore, unnecessary imaging studies that are ordered cause a significant cost to the healthcare system as well as a time cost to the patient, the radiologist, etc. With a paradigm shift from a volume standard to a value standard, the exemplary embodiments provide a mechanism to address the issue of ordering unnecessary imaging studies by reviewing recommendations made by radiologists. That is, when an unnecessary imaging study is ordered, the imaging study may be avoided when a prior imaging study exists that is sufficient to serve the purpose of ordering the unnecessary imaging study. US 2016/147946 A1 discloses a system that facilitates the presentation and comparison of clinical information. However, there is no suggestion of using anything equivalent to a "base" model once the personalization is performed.

### Summary

The invention is defined by the appended claims. The exemplary embodiments are directed to a method, comprising: at an optimization server: receiving a request for relevant prior studies for a patient from a practitioner device utilized by a medical professional, the request including a current study for the patient, the relevant prior studies being relevant to the current study; determining the relevant prior studies from prior studies of the patient based on a personalized model, the personalized model associated with the medical professional, the personalized model indicating a relevance score of the relevant prior studies to the current study; and transmitting the relevant prior studies to the practitioner device.

The exemplary embodiments are directed to an optimization server, comprising: a transceiver communicating via a communications network, the transceiver receiving a request for relevant prior studies for a patient from a practitioner device utilized by a medical professional, the request including a current study for the patient, the relevant prior studies being relevant to the current study; and a processor determining the relevant prior studies from prior studies of the patient based on a personalized model, the personalized model associated with the medical professional, the personalized model indicating a relevance score of the relevant prior studies to the current study, wherein the transceiver transmits the relevant prior studies to the practitioner device.

The exemplary embodiments are directed to a method, comprising: at an optimization server: receiving a report from a practitioner device utilized by a medical professional for a current study for a patient, the report including a recommendation for a further procedure to be performed on the patient; determining relevant prior studies from prior studies of the patient based on a model, the model indicating a relevance score of the relevant prior studies to the current study; determining whether the relevant prior studies negate a need for the recommendation; and when the need for the recommendation is negated, updating the report to remove the recommendation.

### Brief Description of the Drawings

Fig. 1 shows a system according to the exemplary embodiments.
Fig. 2 shows an optimization server of Fig. 1 according to the exemplary embodiments.
Fig. 3 shows a method for generating a base model to generate results of prior studies according to the exemplary embodiments.
Fig. 4 shows a method for updating a personalized model to generate results of prior studies according to the exemplary embodiments.
Fig. 5 shows a method for analyzing a report using results of prior studies according to the exemplary embodiments.

### Detailed Description

The exemplary embodiments may be further understood with reference to the following description and the related appended drawings, wherein like elements are provided with the same reference numerals. The exemplary embodiments are related to a device, a system, and a method for optimizing usage of prior studies, particularly imaging studies. The prior studies may provide pertinent information for analyzing a current study and preparing a report of the results of the current study. However, there may be prior studies that are relevant to the current study while other prior studies may be irrelevant to the current study. The exemplary embodiments provide an automated mechanism in which relevant prior studies are identified. In a first aspect, the relevant prior studies may be provided to a medical professional for review to prepare a report for the current study, from which the manner in which the relevant prior studies are identified may be personalized to the medical professional. In a second aspect, the relevant prior studies may be used to evaluate a recommendation included in a report that may be redundant or unnecessary.

Initially, it is noted that the use of imaging studies and more generally to a radiological workflow associated with imaging studies is only exemplary. As will become apparent below to those skilled in the art, the exemplary embodiments may be modified for use with any workflow in which prior studies or prior documentation may be utilized in processing a current study or a current analysis. Thus, the radiological workflow and associated studies may represent any workflow and any associated documentation.

The exemplary embodiments provide a mechanism that automatically detects prior studies that are likely relevant for use with a current study. Accordingly, the exemplary embodiments are configured to provide a direct positive impact on the efficiency and workload of the radiologist. Furthermore, the exemplary embodiments sort through the prior studies and may rank them according to its relevance to further add to the efficiency of the radiology workflow. Thus, when the radiologist queries a component according to the exemplary embodiments with, for example, the current study and optional comparison criteria, the component may extract all available prior studies associated with the current study, rank them, and return only the relevant prior studies to the radiologist. To properly define how the relevance is identified, the exemplary embodiments may incorporate a comparison criterion that allows for identification of relevant prior studies for a given context using a significant number (e.g., thousands) of relevant and non-relevant study pairs. As any manual approach for this significant number of study pairs is an impossibility, the mechanism according to the exemplary embodiments automatically labels the study pairs and allows for development of a system that is customized to a given institution or radiologist. Using a learning feature, the exemplary embodiments may further personalize the manner in which the results of the prior studies are returned so that the results are customized to the radiologist or institution. As will be described in further detail below, a base model may be created to generate the results of the prior studies for a query, generate ground truths from archived data, and adapt the base model into a personalized model according to behavior data.

The exemplary embodiments may be utilized for imaging studies and associated images as stored and provided through a Picture Archive and Communications System (PACS) or imaging system workstation. The PACS is a workstation that aids radiologists in their duties and allows them to keep up with ever increasing workloads. In particular, the PACS employs an intuitive graphical user interface that provides access to the patient's radiological history, including diagnostic reports, exam notes, clinical history, and imaging scans. Further, the PACS has several features that simplify and speed up workflow. These features are critical in improving the radiologist's productivity. The exemplary embodiments may be utilized in any PACS such as for example Philips Intellispace PACS. The exemplary embodiments may also be utilized in other applications or platforms that use prior studies, such as for example Philips Uronav (prior biopsies and fusion workflow), DynaLync, and Intellispace Portal.

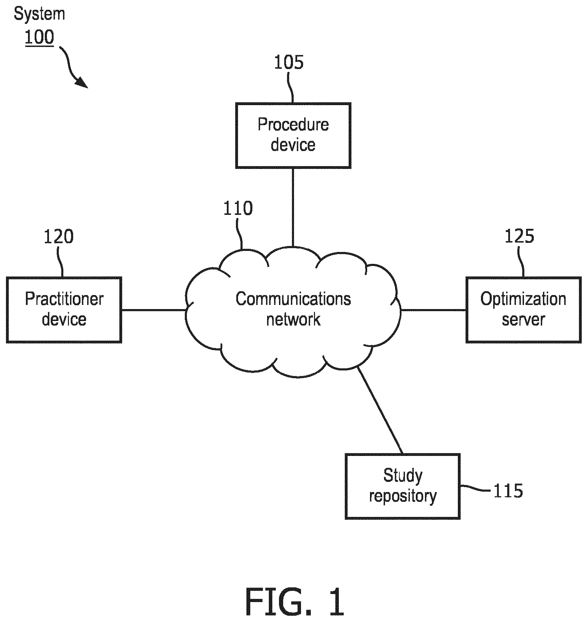

Fig. 1 shows a system 100 according to the exemplary embodiments. The system 100 includes a communication between various components involved in utilizing prior studies of a patient. Specifically, the system 100 includes for example a procedure device 105, a communications network 110, a study repository 115, a practitioner device 120, and an optimization server 125. As will be described in further detail below, the system 100 according to the exemplary embodiments incorporates an entirety of a process in which the prior studies of the patient are identified and utilized.

The procedure device 105 may represent any electronic device that is configured to perform a procedure on the patient. For example, the procedure device 105 may be used for a radiological procedure such as an X-ray scan, a magnetic resonance imaging (MRI) scan, a computerized axial tomography (CAT) scan, etc. Accordingly, the procedure device 105 may include the necessary hardware, software, and/or firmware to perform the various procedures and/or treatments using a specified modality on a body part of the patient. Those skilled in the art will understand that the procedure device 105 may be configured to operate automatically, manually, or a combination thereof. For example, the procedure may be performed by the procedure device 105 automatically from the radiologist or technician initiating the procedure. Thereafter, the other parts of the procedure may be performed in an automated manner. In another example, the procedure may be performed by the procedure device 105 in which the radiologist or technician must continuously provide inputs for actions to be taken in the procedure. The procedure device 105 may also include any connectivity hardware, software, and/or firmware for data to be communicated to another electronic device.

The procedure device 105 may accordingly generate results of the procedure for a user to read. For example, the procedure device 105 may generate images of tissue according to the modality of the procedure device 105 for a selected body part. That is, imaging studies may be generated by the procedure device 105. In a particular example, when the procedure device 105 performs a MRI scan, a corresponding imaging study in which fluid and tissue of the patient is represented in different shades of black may be produced reflecting a condition of the patient at the time the image was captured. In another particular example, when the procedure device 105 performs a CT scan, a corresponding imaging study in which a plurality of X-ray images taken at different angles are used to produce a cross-sectional image of a specific area of a scanned object may be produced reflecting a condition of the patient at the time the image was captured. Thus, depending on the type, the procedure device 105 may generate a corresponding imaging study of the patient including one or more images captured for the study.

The images captured by the procedure device 105 may include or be associated with identification information corresponding to the patient. For example, the patient may be associated with an identification number to uniquely identify the patient. When the procedure device 105 is used for the patient, the resulting images may include or be associated with the identification number. Thus, when images of the patient are to be identified, the identification number may be utilized for this search.

It should also be noted that the system 100 illustrating a single procedure device 105 is only exemplary. Instead, the procedure device 105 may represent one or more procedure devices that are configured to exchange data with the other components of the system 100. For example, the procedure device 105 may represent a set of procedure devices 105 of a hospital that perform procedures and provide corresponding images.

It should also be noted that while the exemplary embodiments have been described as the procedure device 105 being related to medical imaging (e.g., MRI, X-ray, etc.) and the corresponding modalities, the procedure device 105 may be any type of device. Other examples of procedure devices include computed tomography (CT), positron emission tomography (PET), and ultrasound.

The communications network 110 may be configured to communicatively connect the various components of the system 100 to exchange data. The communications network 110 may represent any single or plurality of networks used by the components of the system 100 to communicate with one another. For example, if the procedure device 105 is used at a hospital or a private practice building, the communications network 110 may include a private network in which the procedure device 105 may initially connect. The private network may connect to a network of an Internet service provider to connect to the Internet. Subsequently, through the Internet, a connection may be established to other electronic devices. The communications network 110 and all networks that may be included therein may be any type of network. For example, the communications network 110 may be a local area network (LAN), a wide area network (WAN), a virtual LAN (VLAN), a WiFi network, a HotSpot, a cellular network (e.g., 3G, 4G, Long Term Evolution (LTE), etc.), a cloud network, a wired form of these networks, a wireless form of these networks, a combined wired/wireless form of these networks, etc.

The study repository 115 may be a component that stores imaging studies along with associated images of the imaging study. For example, the study repository 115 may be a PACS using a database that maintains the imaging studies in an electronic format. Accordingly, the procedure device 105 that captures the images and generates the imaging study may transmit this data via the communications network 110 for storage in the study repository 115. The study repository 115 may store the received data in the database in a predetermined arrangement such as by identification numbers of the patients and/or by dates in which the imaging studies were generated or the procedures that captured the images were performed.

The study repository 115 may be configured with a search functionality that allows a radiologist to query the study repository 115 to return any image or imaging study associated based on an input entered for a search parameter. For example, a user may request that imaging studies of a particular patient be returned. Accordingly, the identification number of the patient or the name of the patient may be provided as the search parameter to the study repository 115. In another example, a user may request a specific imaging study or a set of imaging studies of a particular patient be returned.
Accordingly, the identification of the patient and a further search term (e.g., a specific procedure performed on a particular date at a particular time, any procedure performed in a selected period of time, etc.) may be provided as the search parameter to the study repository 115. As will be described in further detail below, the optimization server 125 may also utilize the search functionality as the results indicate all data matching the entered input.

The practitioner device 120 may represent any electronic device that is configured to perform the functionalities corresponding to use associated with a healthcare provider such as a radiologist. For example, the practitioner device 120 may be a portable device such as a tablet, a laptop, etc. or a client stationary device such as a desktop terminal. The practitioner device 120 may include the necessary hardware, software, and/or firmware to perform the various operations associated with medical treatment. The practitioner device 120 may also include the required connectivity hardware, software, and firmware (e.g., transceiver) to establish a connection with the communications network 110 to further establish a connection with the other components of the system 100. For example, the practitioner device 120 may schedule appointments for patients using a calendar application and may track treatments or procedures of a patient, etc. In another example, the practitioner device 120 may be used to generate a report for a current study. Thus, the radiologist may query for the current imaging study as well as any prior imaging studies used in generating the report.

In a substantially similar manner as the procedure device 105, the system 100 illustrating a single practitioner device 120 is only exemplary. Instead, the practitioner device 120 may represent one or more practitioner devices that are configured to exchange data with the other components of the system 100 via the communications network 110. For example, the practitioner device 120 may represent a set of practitioner devices used by practitioners of a hospital.

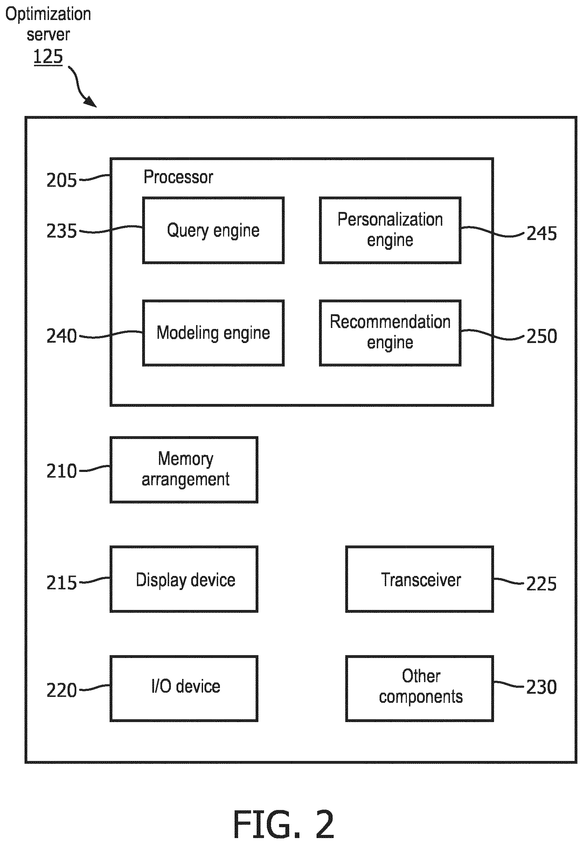

As described above, the optimization server 125 may be a component of the system 100. Specifically, the optimization server 125 may perform functionalities associated with identifying and/or utilizing prior studies. Fig. 2 shows the optimization server 125 of Fig. 1 according to the exemplary embodiments. The optimization server 125 may provide various functionalities associated with the prior studies. Although the optimization server 125 is described as a network component (specifically a server), the optimization server 125 may be embodied in a variety of ways such as a portable device (e.g., a tablet, a smartphone, a laptop, etc.), a client stationary device (e.g., a desktop terminal), incorporated into the procedure device 105 and/or the physician device 120, etc. The optimization server 125 may include a processor 205, a memory arrangement 210, a display device 215, an input and output (I/O) device 220, a transceiver 225, and other components 230 (e.g., an imager, an audio I/O device, a battery, a data acquisition device, ports to electrically connect the optimization server 125 to other electronic devices, etc.).

The processor 205 may be configured to execute a plurality of applications of the optimization server 125. As will be described in further detail below, the processor 205 may utilize a plurality of engines including a query engine 235, a modeling engine 240, a personalization engine 245, and a recommendation engine 250. The query engine 235 may process requests from the practitioner device 120. As will be described in detail below, the requests may relate to providing prior studies having relevance to a current study or analyzing a report and any recommendation included therein. The modeling engine 240 may generate models used in identifying relevant prior studies based on a current study and/or other parameters. The personalization engine 245 may utilize feedback data from the practitioner device 120 to update the models in a personalized manner. The recommendation engine 250 may analyze the report for recommendations.

It should be noted that the above noted applications and engines each being an application (e.g., a program) executed by the processor 205 is only exemplary. The functionality associated with the applications may also be represented as components of one or more multifunctional programs, a separate incorporated component of the optimization server 125 or may be a modular component coupled to the optimization server 125, e.g., an integrated circuit with or without firmware.

The memory 210 may be a hardware component configured to store data related to operations performed by the optimization server 125. Specifically, the memory 210 may store data related to the received requests and studies used in identifying relevant prior studies. The memory 210 may also store data related to the report that is analyzed for recommendations. The display device 215 may be a hardware component configured to show data to a user while the I/O device 220 may be a hardware component that enables the user to enter inputs. For example, an administrator of the optimization server 125 may maintain and update the functionalities of the optimization server 125 through user interfaces shown on the display device 215 with inputs entered with the I/O device 220. It should be noted that the display device 215 and the I/O device 220 may be separate components or integrated together such as a touchscreen. The transceiver 225 may be a hardware component configured to transmit and/or receive data via the communications network 110.

According to the exemplary embodiments, the optimization server 125 may utilize an identification functionality in which relevant prior studies are identified and utilize the relevant prior studies for various different operations. Initially, as described above, the query engine 235 may process requests from the practitioner device 120. Thus, the practitioner device 120 may connect to the optimization server 125. It is noted that the connection that is established may be a direct or indirect connection. For example, the practitioner device 120 may have an application installed thereon that enables a connection to the optimization server 125 to be established. In another example, the practitioner device 120 may establish a connection to the study repository 115. However, the system 100 may be configured such that the optimization server 125 performs its functionality as an intermediary or prior to any connection with the study repository 115. It is again noted that the use of a separate optimization server 125 is only exemplary. For example, the optimization server 125 may provide functionalities in an application that is installed on the practitioner device 120. Accordingly, a direct connection may still be established with the study repository 115.

The requests from the practitioner device 120 may relate to utilizing relevant prior studies. As noted above, a first manner of relevant prior study use is when the request from the practitioner device 120 is directly for providing the relevant prior studies to the practitioner device 120. For example, the radiologist may be reviewing a current imaging study and would like to include a tracking of a diagnosis for a detected condition in a report. Accordingly, prior imaging studies may be reviewed to include the tracking information in the report. Thus, the radiologist may submit a request via the practitioner device 120 for the prior imaging studies, particularly those that are relevant to the current reason for submitting the request. Also noted above, a second manner in which relevant prior studies is utilized is when a report is analyzed for any recommendation included therein. For example, the radiologist may have generated a report with a recommendation for a further procedure for the patient as more information may be required from the current imaging study. Accordingly, prior imaging studies may be reviewed to determine whether more information may already be available to re-assess whether the recommendation is still warranted.

To identify the prior studies that are relevant or assign a relevancy value to a prior study for the request, the exemplary embodiments may utilize a model. As noted above, the modeling engine 240 may generate models used in identifying relevant prior studies based on a current study and/or other parameters. Thus, the query engine 235 may operate in conjunction with the modeling engine 240 to generate a response to the request received from the practitioner device 120.

Initially, the modeling engine 240 may be configured to generate a base model. The base model may be independent of the request and any other search parameters. Specifically, a ground truth may be established for the prior studies. To construct machine learning models, the modeling engine 240 may identify pairs of studies and determine the relevancy for these pairs. Those skilled in the art will understand that the base model may be used in a more efficient manner with a larger number of pairs of studies (e.g., thousands of pairs of studies). Accordingly, the modeling engine 240 provides an automated approach to pairing the studies and determining the relevancy to one another.

The modeling engine 240 may generate the base model by first collecting existing studies. For example, the studies may be retrieved from the study repository 115 that may be, for example, a PACS, a radiology information system (RIS), etc. A more exhaustive base model may be generated when a greater number of studies are used to generate the model. After collecting the studies, the modeling engine 240 may sort the studies in preparation for subsequent processing. For example, the studies may be sorted by date and/or time. It is noted that the prior studies are not limited in any way to modality or body part. That is, a semantic approach is not required to be utilized by the modeling engine 240. Instead, any prior study may be utilized to identify a relevance, independent of the modality that was used in the study and/or the body part being imaged in the study.

In an exemplary manner in which studies (S) are paired and a relevancy therebetween is determined, the modeling engine 240 may list all possible pairs of studies. For example, the list L may be defined with L = [(S₁, S₂), (S₁, S₃)...(Sₙ-₁, Sₙ)].
Each study pair (Sₖ, Sⱼ) is such that study Sₖ is older in time than study Sⱼ. Each study pair (Sₖ, Sⱼ) is also such that there is a ground truth (GT) label Rₖ associated with study Sₖ to determine relevance. When a GT label is directly extracted, for each study pair (Sₖ, Sj), the modeling engine 240 determines whether there is a direct or indirect reference study Sⱼ that is associated with study Sₖ. The modeling engine 240 may utilize various different mechanisms to perform this determination such as natural language processing (NLP), machine learning, etc. If a reference is determined, then the study pair (Sₖ, Sⱼ) is labelled as relevant. However, if a reference is not determined, then the study pair (Sₖ, Sⱼ) is labelled as not relevant.

Furthermore, a relevance score may also be determined such that a relative relevance parameter may be determined for each study pair that is labelled as relevant. Thus, relevant prior imaging studies may be ranked based on the corresponding relevance scores. Accordingly, when operating in conjunction with the query engine 235, the most relevant prior imaging studies may be identified with respect to the current study, for example, using the base model.

Specifically, the modeling engine 240 may generate the base model that implements a statistical model that estimates the relevancy of study pairs as a probability score. The base model may comprise a feature extractor and a statistical model. It is noted that any statistical model that estimates the probability of relevance of a current study to a prior study pair individually or jointly for an entire sequence of study pairs may be used. For example, the statistical models may be a logistic regression, a random forest, a support vector machine, a maximum entropy Markov model, a hidden Markov model, a conditional random field, etc.

The statistical model may also use information extracted by the feature extractor which is configured to extract both explicit and implicit features. The explicit features may include direct encoding of any data contained in study meta-data (e.g., Digital Imaging and Communications in Medicine (DICOM) tags). The implicit features may represent the encoding of information derived from meta-tags. For example, the implicit features may include anatomy, modality, anomalies, condition, history, etc. that are inferred from several DICOM metadata fields. In another example, the implicit features may include a cross product of study pair modalities, anatomies, study reasons, procedure description etc., either individually or together. In a further example, the implicit features may include a likelihood of relevance estimated using statistical distribution models based on a time difference between studies across modalities, anatomies, reason for studies, or individually.

Using the above mechanism, the modeling engine 240 may generate the base model which is used to identify relevant prior studies in regard to a current study and other search parameters. As the available prior studies (e.g., in the study repository 115) may increase over time, the modeling engine 240 may update the base model accordingly. For example, for each additional prior study that is added, the modeling engine 240 may update the base model. In another example, the modeling engine 240 may update the base model after a predetermined number of new prior studies are added. In a further example, the modeling engine 240 may update the base model after a predetermined amount of time to incorporate any new prior study that may have been added.

The base model may provide an initial manner in which the relevant prior studies are identified. However, as those skilled in the art will understand, each radiologist or each medical organization/facility (e.g., hospital) may utilize different criteria or have different preferences in identifying relevance in prior studies. For example, for a current study of a patient, a first radiologist may regard a first prior study to be of high relevance while a second prior study is of lower relevance. A second radiologist may use an opposite configuration in which the first prior study is regarded with intermediate relevance while the second prior study is of higher relevance. Thus, the personal preferences of the radiologist may affect the manner in which prior studies are regarded in terms of relevance. Accordingly, the base model may be used to generate the same set of results for a given set of parameters (e.g., a current study) but radiologists receiving the results may utilize the results in different manners.

To accommodate personal preferences of the radiologists, as noted above, the personalization engine 245 may utilize feedback data from the practitioner device 120 to update the models in a personalized manner. Specifically, the base model may be updated to a personalized model and the personalized model may be continuously updated to reflect the personal preferences of the radiologist. The feedback data used by the personalization engine 245 may be the manner in which previously provided results are used by the radiologist. For example, the radiologist may transmit a request for relevant prior studies with respect to a current study. The optimization server 125 may utilize the base model (if no personalized model exists for the radiologist) or the personalized model for the radiologist. The results based on the available model may be transmitted back to the radiologist. The radiologist may utilize selected prior studies in the results for the current study. The inputs associated with the use of the results may be provided to the personalization engine 245. In this manner, the personalization engine 245 may analyze the manner in which the radiologist performs radiological workflows and customize the available model to the preferences of the radiologist. The personalization engine 245 may customize the model such that the results that are labelled relevant/non-relevant may be updated and/or the relevancy value assigned to relevant prior studies may be updated.

In a specific implementation of customizing the model in which relevant prior studies are identified, a radiologist may transmit a query comprising of a current study and optional comparison criteria. Assuming that the radiologist is a user who is utilizing the feature of the optimization server 125 for the first time and a personalized model does not exist for the radiologist, the optimization server 125 may produce a list of relevant prior studies using the base model. The radiologist may review the list of relevant prior studies which may be ordered based on relevancy values (as defined by the base model). The radiologist may select the relevant prior studies that are to be further reviewed. The personalization engine 245 may receive the inputs of the radiologist as feedback data. This feedback data may be used to update the base model according to the selections and the list. For example, a lazy learning or a case-based learning may be used.

The feedback data may relate to a variety of different inputs by the radiologist. For example, the feedback data may relate to a radiologist's choice in terms of the reason for seeking relevance. In a first example, the reason may be for a comparison (e.g., CT time point 1 relative to CT time point 2). In a second example, the reason may be for a better resolution (e.g., CT 1.0 mm spacing relative to CT 0.5 mm spacing). In a third example, the reason may be for complimentary information (e.g., CT relative to PET). Depending on the radiologist's selection, different criteria may be considered to update the base model and create a personalized model that matches the personal preferences of the radiologist. For example, the radiologist may always ignore prior studies that are outdated by a predetermined minimum amount of time. The personalized model may be updated to decrease the relevance or eliminate the prior studies that go beyond this predetermined minimum amount of time. Furthermore, the radiologist may be allowed to assign priorities to determine relevance based on the radiologist's preference (e.g., modality, anatomy, finding, time, etc.). In this manner, the optimization server 125 may utilize relevant prior studies that are defined by a personalized model which incorporates radiologist preferences to a base model.

As described above, the modeling engine 240 may continue to update the base model with further prior studies being added. The personalized model may also be updated in a substantially similar manner. For example, the base model may initially be updated. Using the feedback data collected for the radiologist, the personalized model may be generated for each updated base model.

Using the above manner of generating a personalized model, the optimization server 125 may be configured to appropriately generate results to a request from the radiologist for prior studies corresponding to the current study and other criteria included in the request. As described above, the optimization server 125 may utilize the prior studies in a first manner in which results including the prior studies are provided to the radiologist. Thus, the radiologist submitting the request may package parameters which are used as the basis for identifying the relevant prior studies. The base model or personalized model associated with the radiologist may be used to identify the relevant prior studies to create a list of the relevant prior studies, organized based on relevance values. The list may be provided back to the radiologist who may review the items of the list (e.g., open an image in the prior study). Thereafter, the radiologist may, for example, create a report for the current study which used the prior studies returned from the request. The report may describe the findings of the current study where the findings are relative to corresponding findings in the prior studies. The report may also identify a subsequent course of action for the patient such as further procedures, particularly if the current study does not provide sufficient information to clarify the reason for performing the current study.

As described above, the optimization server 125 may utilize prior studies for other purposes. In the second manner described above, the optimization server 125 may utilize the recommendation engine 250 to analyze a report that is created for a current study for recommendations. It is noted that the description herein relates to a medical report indicative of results of an imaging study. However, the exemplary embodiments may be utilized for other types of reports. For example, the exemplary embodiments may also be utilized for other types of reports such as a surgery report, an interventional cardiology, a pathology report, an admission note, a progress note, a discharge note, etc.

The recommendation engine 250 may utilize various modules in providing this functionality. In a first module, the recommendation engine 250 may include a recommendation detection module that detects recommendation-related phrases. The report by the radiologist may be generated with various modes. For example, the radiologist may prefer to type out the report. In another example, the radiologist may dictate and record the report with speech. Accordingly, the recommendation detection module may be configured to accommodate text extraction and/or speech detection. With text extraction, the recommendation detection module may include a NLP pipeline that parses sentences and extracts the term "recommend" or similar phrases (e.g., "suggested"). With speech detection, the recommendation detection module may detect the term "recommend" or similar phrases from speech. The detection of the terms in text or speech may be performed in real time such that the further operations may be performed immediately, even before the report has been completed. However, the performance in real time is only exemplary and the recommendation detection module may be utilized after the report has been created.

In a second module, the recommendation engine 250 may include a modality and imaging site detection module. Specifically, the modality may relate to the type of procedure used in capturing the image of the current study. The imaging site may relate to the body part that is targeted for imaging. In a substantially similar manner as the recommendation detection module, the modality and imaging site detection module may be configured for text and speech. With text extraction, the modality and imaging site detection module may again utilize the NLP pipeline to parse sentences to extract the modality and body part. With speech detection, the modality and imaging site detection module may detect phrases describing the modality as well as the body part. The modality and imaging site detection module may also operate in real time.

In a third module, the recommendation engine 250 may include a relevant prior imaging study detection module. The relevant prior imaging study detection module may be equivalent to the modeling engine 240 and/or the personalization engine 245. Specifically, the relevant prior imaging study detection module may generate a base model or update the base model as a personalized model that is used identify relevant prior studies and assign relevance values with respect to the current study and other criteria.

In a fourth module, the recommendation engine 250 may include a feedback interface module. The feedback interface module may provide a user interface to provide feedback to the radiologist when a relevant prior study is detected. Using the information determined with the recommendation detection module, the modality and imaging site detection module, and the relevant prior imaging study detection module, the feedback interface module may determine if any of the relevant prior studies identified by the relevant prior imaging study detection module addresses the recommendation and modality/imaging site identified by the recommendation detection module and the modality and imaging site detection module, respectively. In this manner, the recommendation and modality/imaging site may be comparable to the current study used in the first manner of utilizing the optimization server 125. The model and relevant prior studies may be substantially similar to the first manner as well. A further functionality in the second manner of utilizing the optimization server 125 therefore entails an automated operation to identify the relevant prior study that potentially contradicts the need for the imaging study that is recommended to be ordered. The feedback interface module may accordingly create a user interface in which the identified relevant prior study is provided to the radiologist for consideration to override the recommendation (e.g., review whether the recommendation is still warranted).

It is noted that the recommendation engine 250 may include further modules. In a first further module, the recommendation engine 250 may include an extraction module that is configured to extract patient relevant history (e.g., medications, implants, allergies, medical conditions such as HIV, diabetes, etc.). If there is a concern with respect to the recommended imaging studies, a warning message may be generated to inform the radiologist to be aware of the consequence of the recommendation to order the imaging study. In a second further module, the recommendation engine 250 may be modified for other purposes such as biopsies or therapy recommendations. Thus, unnecessary biopsies or therapy recommendations may be detected using prior studies. An alert may be generated if the biopsy is already detected in a relevant prior study or of potential harm in conducting a therapy recommendation due to the patient's history.

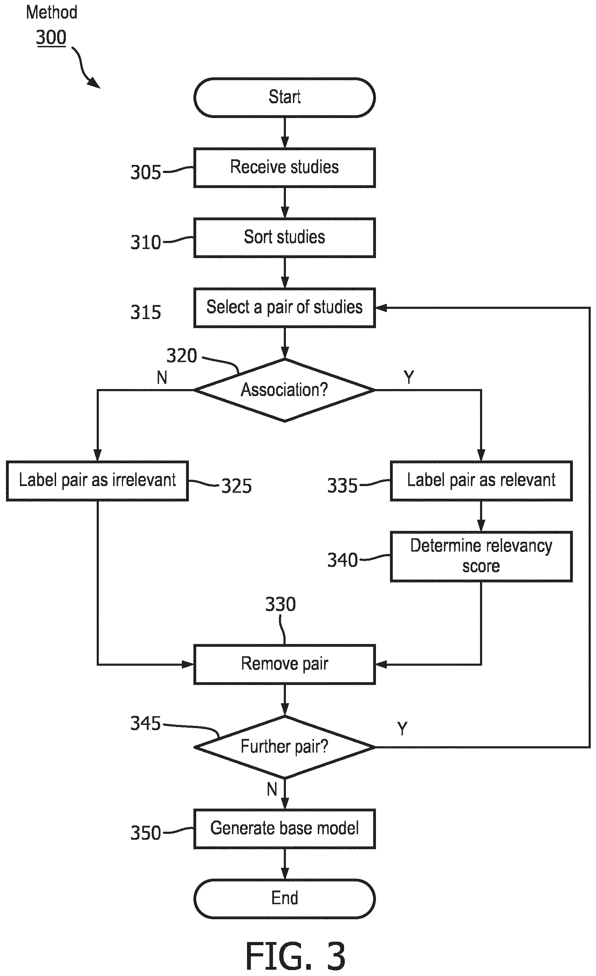

Fig. 3 shows a method 300 for generating a base model to generate results of prior studies according to the exemplary embodiments. Specifically, the method 300 may relate to an operation performed so that the first and second manners of utilizing the relevant prior studies may be used. Accordingly, the method 300 will be described from the perspective of the optimization server 125. The method 300 will also be described with regard to the system 100 of Fig. 1 and the plurality of engines 235-245 of the optimization server 125 of Fig. 2.

In step 305, the optimization server 125 receives prior studies. For example, the optimization server 125 may receive the prior studies stored in the study repository 115, such as PACS, RIS, etc. The optimization server 125 may be configured to retrieve all prior studies that are available to perform the functionalities herein. In step 310, the optimization server 125 may sort the prior studies. As described above, the optimization server 125 may sort the prior studies based on date and time such that the prior studies are listed in chronological order.

In step 315, the optimization server 125 selects a pair of studies. As described above, two studies may be selected in which one of the studies is later in time than the other of the studies. The studies may further have a ground truth label associated therewith to determine a relevance. The relevance may be determined using any of the above described methods. Thus, in step 320, the optimization server 125 determines whether there is a relevancy association with the pair.

If there is no relevancy with the pair (i.e., the pair is irrelevant to one another), the optimization server 125 continues the method 300 to step 325. In step 325, the optimization server 125 labels the pair as irrelevant. Then, in step 330, the optimization server 125 removes the pair from consideration. That is, the pairing is removed, not the individual studies in the pair.

If there is a relevancy with the pair, the optimization server 125 continues the method 300 to step 335. In step 335, the optimization server labels the pair as relevant. Furthermore, in step 340, the optimization server 125 determines a relevancy score for the pair. For example, the relevancy score may be based on a probability score in which information extracted with a feature extractor is used by a statistical model to determine the probability score. Then, in step 330, the optimization server 125 removes the pair from consideration.

In step 345, the optimization server determines whether there are any further pairs of studies. If there is at least one further pair of studies, the optimization server 125 returns the method 300 to step 315. However, if the optimization server 125 has analyzed each pair of studies, in step 350, the optimization server 125 generates the base model.

It is noted that the model may be generated and/or updated at a variety of times. As described above, the model may be updated when additional prior studies are added. Thus, in a first example, the model may be updated when any prior study is determined to have been added. Thus, the model may incorporate this new prior study. In a second example, the model may be updated after a predetermined number of new prior studies are determined to have been added. Thus, the model may incorporate these new prior studies. In a third example, the model may be updated after a predetermined amount of time. Thus, the model may incorporate any added new prior studies. In a fourth example, the model may be updated when the features of the optimization server 125 are requested by a radiologist via the practitioner device 120. Thus, the model may incorporate all available prior studies at the time the request is received.

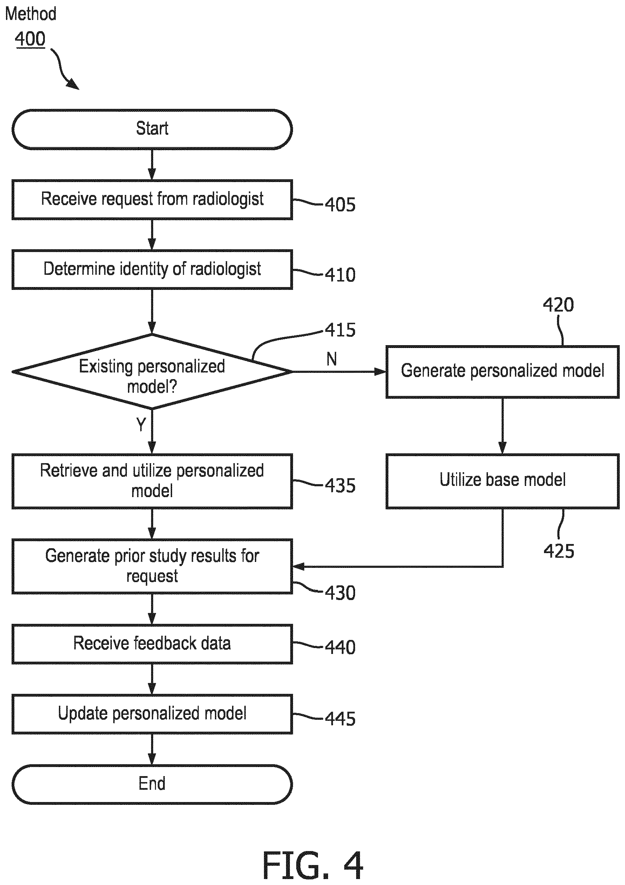

Fig. 4 shows a method 400 for updating a personalized model to generate results of prior studies according to the exemplary embodiments. Specifically, the method 400 may relate to an operation that is performed using the models created from the method 300 as well as generating a personalized model. Accordingly, the method 400 will be described from the perspective of the optimization server 125. The method 400 will also be described with regard to the system 100 of Fig. 1 and the plurality of engines 235-245 of the optimization server 125 of Fig. 2.

In step 405, the optimization server 125 receives a request from the radiologist using the practitioner device 120. As described above, the request may include a current study and other criteria. The request may also relate to receiving results of relevant prior studies for the entered or attached inputs of the request. The request may further include an identification of the radiologist or the practitioner device 120 from which the request is received. In step 410, the optimization server determines an identity of the radiologist from which the request originates.

In step 415, the optimization server 125 determines whether the radiologist has an existing personalized model associated therewith. As described above, the results of relevant prior studies may be determined with a model and the model may be a personalized model which incorporates personal preferences specific to a radiologist. It is noted that personalized models that have been created for the various radiologists may be stored in various locations. In a first example, the personalized models may be stored in the memory arrangement 210 of the optimization server 125. In a second example, the personalized models may be stored in a network repository or separate component. In a third example, the personalized models may be individually stored in a memory arrangement of the practitioner device 120. Thus, the request may include the personalized model or the optimization server 125 may request that the personalized model be transmitted (e.g., as a background operation after receiving the request).

If the radiologist does not have a personalized model associated thereto, the optimization server 125 continues the method 400 to step 420. In step 420, the optimization server 125 generates a personalized model that is associated with the radiologist. In step 425, the optimization server 125 utilizes the base model. For example, the base model may have been created from the process described with respect to the method 300. As no feedback data is available for the radiologist, at this stage of the process, the personalized model may be the same as the base model. Thus, in step 430, the optimization server 125 generates the results of the prior studies for the request based on the currently created personalized model which is the same as the base model. The prior studies may also have a relevance score determined for the request based on the currently created personalized model.

Returning to step 415, if the radiologist already has a personalized model associated thereto, the optimization server 125 continues the method 400 to step 435. In step 435, the optimization server 125 retrieves and utilizes the personalized model which is modified from the base model with feedback data related to previous times that the radiologist has utilized the features of the optimization server 125. Thus, in step 430, the optimization server 125 generates the results of the prior studies for the request based on the already existing personalized model. The prior studies may also have a relevance score determined for the request based on the already existing personalized model.

Te optimization server 125 may have transmitted the results back to the practitioner device 120. The results may be ordered according to the relevance score. The results may also include a particular number of prior studies based on a predetermined standard. In a first example, the optimization server 125 may include only those prior studies that satisfy a minimum relevance score threshold. In a second example, the optimization server 125 may include the top predetermined number of prior studies based on relevance score. Accordingly, a fixed number of results are provided (unless the number of results founds does not satisfy the top predetermined number). In a third example, a combination of the first and second examples may be used. Accordingly, the minimum relevance score threshold may be dynamically adjusted if the number of results for the first example do not satisfy the top predetermined number of prior studies for the second example (e.g., the minimum relevance score threshold may be lowered to reach the top predetermined number).

Upon receiving the results of the request, the radiologist may utilize the prior studies as the radiologist sees fit. In the course of utilizing the prior studies of the results, feedback data of the manner in which the radiologist used the results is gathered. In step 440, the optimization server 125 receives the feedback data. In step 445, the optimization server 125 updates the personalized model based on the feedback data. Thus, in any subsequent request from the radiologist, the personalized model that incorporates the personal preferences of the radiologist may be utilized in determining the results identifying relevant prior studies for the request.

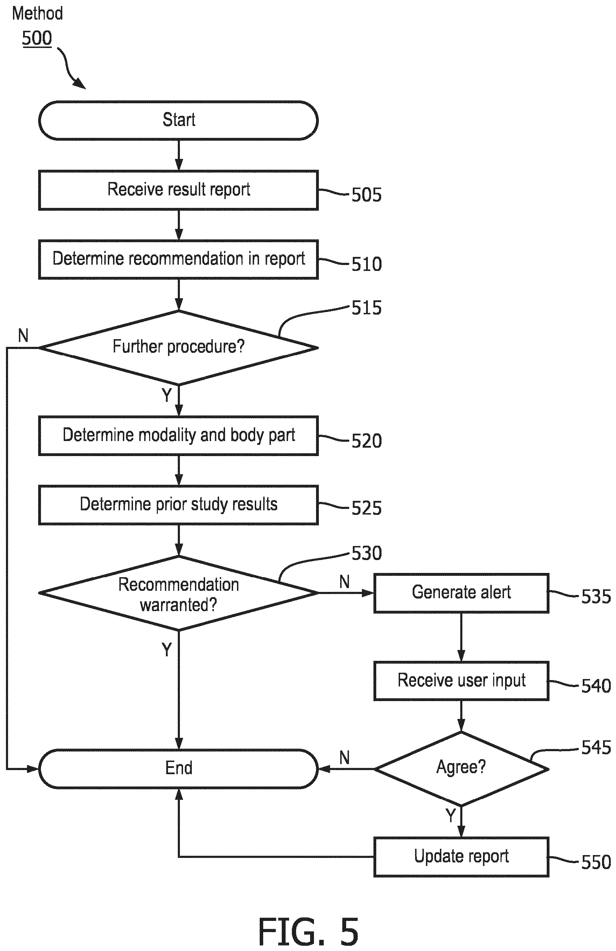

Fig. 5 shows a method 500 for analyzing a report using results of prior studies according to the exemplary embodiments. Specifically, the method 500 may relate to an operation that is performed using the models created from the method 300 while a report is being generated. Accordingly, the method 500 will be described from the perspective of the optimization server 125. The method 500 will also be described with regard to the system 100 of Fig. 1 and the plurality of engines 235-250 of the optimization server 125 of Fig. 2.

In step 505, the optimization server 125 receives the result of the report being generated by the radiologist. As described above, the report may be generated by the radiologist in a variety of different ways. In a first example, the radiologist may prefer to utilize text in which the report is written. In a second example, the radiologist may prefer to dictate the report and record an audio session with speech.

In step 510, the optimization server 125 determines a recommendation included in the report. As described above, a NLP pipeline may be used in parsing the text or identifying target words or phrases indicative of a recommendation. Thus, in step 515, the optimization server 125 determines whether a further procedure is ordered or recommended. If no further procedure is ordered, no recommendation is required to be analyzed. Thus, the method 500 may end. It is noted that the further procedure is only exemplary and the optimization server 125 may also determine whether a biopsy or a therapy recommendation is included in the report.

If a further procedure is ordered, in step 520, the optimization server 125 determines a modality and body part identified in the report. In a substantially similar manner as identifying a recommendation in the report, the modality and body part may also utilize the NLP pipeline to identify the modality that is used in the current imaging study and the body part captured in the image of the current imaging study to which the report is being created.

In step 525, the optimization server 125 determines the prior studies that are relevant to the report, the recommendation in the report, and the modality/body part identified for the report. As described above, the optimization server 125 may utilize a model with the extracted information to identify the relevant prior studies. The model that is selected for use in this process may be either the base model or the personalized model. For example, the base model may provide a standardized approach in which the recommendations are reviewed with a consistent manner. In another example, the personalized model may provide a personal approach in which the recommendations are reviewed incorporating the personal preferences of the radiologist in utilizing relevant prior studies.

In step 530, the optimization server 125 determines whether the recommendation that is detected in the report is warranted, particularly given the relevant prior studies that have been identified. As the recommendation is for a procedure being ordered for the patient (e.g., the current imaging study with the prior studies that were also reviewed are insufficient), the optimization server 125 may utilize the results of the identification of prior studies in step 525 to determine if the recommendation is unnecessary. If the optimization server 125 determines that the recommendation is necessary, the method 500 ends.

It is noted that even if the same model is used for identifying prior studies used in creating the report as well as for identifying prior studies in step 525, the results of the prior studies may not be identical. For example, the modality and body part identified in both scenarios may be different from the different mechanisms used in their identification. In fact, the method 500 may be performed utilizing the base model so that a different model is used and the results are more likely to be different from the results from using the personalized model.

Returning to step 530, if the prior studies indicate that the recommendation is not warranted, the optimization server 125 continues the method 500 to step 535. In step 535, the optimization server 125 generates an alert in a user interface. Specifically, the optimization server 125 indicates that the recommendation should be reviewed again particularly in light of an identified relevant prior study. The radiologist may be provided an opportunity to review the identified relevant prior study.

In step 540, the radiologist provides a user input indicating whether the identified relevant prior study indeed negates the need for the recommendation. Thus, in step 545, if the radiologist disagrees with the analysis to overturn the recommendation, the method 500 ends. However, if the radiologist agrees with the analysis to overturn the recommendation, in step 550, the report is updated accordingly to remove the recommendation. It is noted that the updating of the report may be manually performed by the radiologist or automatically performed by the optimization server 125.

It should again be noted that the above description of the exemplary embodiments is described with a radiological workflow with associated imaging studies and the use of the radiological workflow is only exemplary. The exemplary embodiments may be utilized with any medical workflow in which prior studies or prior documentation is utilized for a current study or preparation of a report. In fact, the use of a medical workflow is only exemplary and the exemplary embodiments may be utilized for any workflow in which prior documentation (not necessarily images) is utilized. Thus, the use of the radiological workflow may represent any scenario in which an efficiency is improved from using prior documentation.

The exemplary embodiments provide a device, system, and method of identifying prior studies that are relevant. The relevant prior studies may be utilized for various reasons. The exemplary embodiments may receive a request from a radiologist for relevant prior studies to be reviewed for a current study. Independent of any modality or body part in the current study, the relevant prior studies may be identified based on models that incorporate a large plurality of prior studies to determine relevance and degree of relevance. The exemplary embodiments may also be configured to review reports and recommendation in reports to determine whether the recommendation is warranted given identified relevant prior studies.

Those skilled in the art will understand that the above-described exemplary embodiments may be implemented in any suitable software or hardware configuration or combination thereof. An exemplary hardware platform for implementing the exemplary embodiments may include, for example, an Intel x86 based platform with compatible operating system, a Windows platform, a Mac platform and MAC OS, a mobile device having an operating system such as iOS, Android, etc. In a further example, the exemplary embodiments of the above described method may be embodied as a computer program product containing lines of code stored on a computer readable storage medium that may be executed on a processor or microprocessor. The storage medium may be, for example, a local or remote data repository compatible or formatted for use with the above noted operating systems using any storage operation.

It will be apparent to those skilled in the art that various modifications may be made in the present disclosure. Thus, it is intended that the present disclosure cover modifications and variations of this disclosure provided they come within the scope of the appended claims and their equivalent.

## Claims

1. A computer-implemented method for identifying prior medical studies of a patient, comprising:
receiving a request for relevant prior studies for a patient from a practitioner device utilized by a medical professional, the request including a current study for the patient, the relevant prior studies being relevant to the current study;
determining the relevant prior studies from prior studies of the patient based on a personalized model, the personalized model associated with the medical professional, the personalized model indicating a relevance score of the relevant prior studies to the current study, wherein the personalized model is based on a base model and feedback data, wherein the base model is created based on the prior studies and further prior studies for further patients; and
receiving the prior studies and the further prior studies, sorting the prior studies and further prior studies;
generating a list of pairs of the prior studies and the further prior studies; and
determining a ground truth label indicative of whether each of the pairs is one of a relevant pair and an irrelevant pair;
transmitting the relevant prior studies to the practitioner device;
receiving a report from the practitioner device for the current study, the report including a recommendation for a further procedure to be performed on the patient;
determining further relevant prior studies from the prior studies of the patient based on the base model;
determining whether the further relevant prior studies negate a need for the recommendation; and
when the need for the recommendation is negated, updating the report to remove the recommendation.

2. The method of claim 1, wherein the base model is further created based on a feature extractor and a statistical model to determine a base relevance score for each of the pairs that is the relevant pair.

3. The method of claim 1, wherein the feedback data includes at least one input received from the practitioner device for a prior request after the prior relevant studies to the prior request are received.

4. The method of claim 1, wherein the recommendation is identified using one of a text extraction and a speech detection based on whether the report is created using one of text and speech.

5. An optimization server, comprising:
a transceiver communicating via a communications network, the transceiver receiving a request for relevant prior studies for a patient from a practitioner device utilized by a medical professional, the request including a current study for the patient, the relevant prior studies being relevant to the current study; and
a processor determining the relevant prior studies from prior studies of the patient based on a personalized model, the personalized model associated with the medical professional, the personalized model indicating a relevance score of the relevant prior studies to the current study, wherein the personalized model is based on a base model and feedback data, wherein the base model is created based on the prior studies and further prior studies for further patients; receiving the prior studies and the further prior studies, sorting the prior studies and further prior studies, generating a list of pairs of the prior studies and the further prior studies, and determining a ground truth label indicative of whether each of the pairs is one of a relevant pair and an irrelevant pair;
wherein the transceiver transmits the relevant prior studies to the practitioner device,
wherein the transceiver further receives a report from the practitioner device for the current study, the report including a recommendation for a further procedure to be performed on the patient, wherein the processor further determines further relevant prior studies from the prior studies of the patient based on the base model, determines whether the further relevant prior studies negate a need for the recommendation, and when the need for the recommendation is negated, updates the report to remove the recommendation.

6. The optimization server of claim 5, wherein the feedback data includes at least one input received from the practitioner device for a prior request after the prior relevant studies to the prior request are received.

7. The optimization server of claim 5, wherein the recommendation is identified using one of a text extraction and a speech detection based on whether the report is created using one of text and speech.

8. A computer program product comprising instructions which, when the program is executed by a computer, cause the computer to carry out the steps of the method of any one of claims 1-4.

## Patentansprüche

1. Computerimplementiertes Verfahren zum Identifizieren von früheren medizinischen Untersuchungen eines Patienten, umfassend:
Empfangen einer Anfrage über relevante frühere Untersuchungen für einen Patienten von einer Arztvorrichtung, die von einem Mediziner genutzt wird, wobei die Anfrage eine aktuelle Untersuchung für den Patienten beinhaltet, wobei die relevanten früheren Untersuchungen relevant für die aktuelle Untersuchung sind;
Bestimmen der relevanten früheren Untersuchungen aus früheren Untersuchungen des Patienten basierend auf einem personalisierten Modell, wobei das personalisierte Modell dem Mediziner zugeordnet ist, wobei das personalisierte Modell eine Relevanzbewertung der relevanten früheren Untersuchungen für die aktuelle Untersuchung angibt, wobei das personalisierte Modell auf einem Basismodell und Rückmeldungsdaten basiert, wobei das Basismodell basierend auf den früheren Untersuchungen und weiterer früherer Untersuchungen für weitere Patienten erstellt wird; und
Empfangen der früheren Untersuchungen und der weiteren früheren Untersuchungen, Sortieren der früheren Untersuchungen und der weiteren früheren Untersuchungen;
Generieren einer Liste von Paaren aus den früheren Untersuchungen und den weiteren früheren Untersuchungen; und
Bestimmen einer Ground-Truth-Kennzeichnung, die angibt, ob jedes der Paare eines von einem relevanten Paar und einem irrelevanten Paar ist;
Übertragen der relevanten früheren Untersuchungen an die Arztvorrichtung;
Empfangen eines Berichts von der Arztvorrichtung für die aktuelle Untersuchung, wobei der Bericht eine Empfehlung für ein weiteres Vorgehen beinhaltet, das an dem Patienten durchzuführen ist;
Bestimmen von weiteren relevanten früheren Untersuchungen aus den früheren Untersuchungen des Patienten basierend auf dem Basismodell;
Bestimmen, ob die weiteren relevanten früheren Untersuchungen eine Notwendigkeit für die Empfehlung negieren; und
wenn die Notwendigkeit für die Empfehlung negiert wird, Aktualisieren des Berichts, um die Empfehlung zu entfernen.

2. Verfahren nach Anspruch 1, wobei das Basismodell weiter basierend auf einem Merkmalsextraktor und einem statistischen Modell erstellt wird, um eine Basisrelevanzbewertung für jedes der Paare, welches das relevante Paar ist, zu bestimmen.

3. Verfahren nach Anspruch 1, wobei die Rückmeldungsdaten mindestens eine Eingabe beinhalten, die von der Arztvorrichtung auf eine frühere Anfrage empfangen wurde, nachdem die früheren relevanten Untersuchungen für die frühere Anfrage empfangen worden sind.

4. Verfahren nach Anspruch 1, wobei die Empfehlung unter Verwendung einer von einer Textextrahierung und einer Spracherkennung darauf basierend identifiziert wird, ob der Bericht unter Verwendung von einem von Text oder Sprache erstellt wird.

5. Optimierungsserver, umfassend:
einen Sendeempfänger, der über ein Kommunikationsnetzwerk kommuniziert, wobei der Sendeempfänger eine Anfrage über relevante frühere Untersuchungen für einen Patienten von einer Arztvorrichtung, die von einem Mediziner genutzt wird, empfängt, wobei die Anfrage eine aktuelle Untersuchung für den Patienten beinhaltet, wobei die relevanten früheren Untersuchungen relevant für die aktuelle Untersuchung sind; und
einen Prozessor, der die relevanten früheren Untersuchungen aus früheren Untersuchungen des Patienten basierend auf einem personalisierten Modell bestimmt, wobei das personalisierte Modell dem Mediziner zugeordnet ist, wobei das personalisierte Modell eine Relevanzbewertung der relevanten früheren Untersuchungen für die aktuelle Untersuchung angibt, wobei das personalisierte Modell auf einem Basismodell und Rückmeldungsdaten basiert, wobei das Basismodell basierend auf den früheren Untersuchungen und weiteren früheren Untersuchungen für weitere Patienten erstellt wird; und der die früheren Untersuchungen und die weiteren früheren Untersuchungen empfängt, die früheren Untersuchungen und die weiteren früheren Untersuchungen sortiert, eine Liste von Paaren der früheren Untersuchungen und der weiteren früheren Untersuchungen generiert und eine Ground-Truth-Kennzeichnung bestimmt, die angibt, ob jedes der Paare eines von einem relevanten Paar und einem irrelevanten Paar ist;
wobei der Sendeempfänger die relevanten früheren Untersuchungen an die Arztvorrichtung überträgt,
wobei der Sendeempfänger weiter einen Bericht von der Arztvorrichtung für die aktuelle Untersuchung empfängt, der Bericht eine Empfehlung für ein weiteres Vorgehen beinhaltet, das an dem Patienten durchzuführen ist, wobei der Prozessor weiter weitere relevante frühere Untersuchungen aus den früheren Untersuchungen des Patienten basierend auf dem Basismodell bestimmt, bestimmt, ob die weiteren relevanten früheren Untersuchungen eine Notwendigkeit für die Empfehlung negieren, und, wenn die Notwendigkeit für die Empfehlung negiert wird, den Bericht aktualisiert, um die Empfehlung zu entfernen.

6. Optimierungsserver nach Anspruch 5, wobei die Rückmeldungsdaten mindestens eine Eingabe beinhalten, die von der Arztvorrichtung auf eine frühere Anfrage empfangen worden ist, nachdem die früheren relevanten Untersuchungen für die frühere Anfrage empfangen worden sind.

7. Optimierungsserver nach Anspruch 5, wobei die Empfehlung unter Verwendung einer von einer Textextrahierung und einer Spracherkennung darauf basierend identifiziert wird, ob der Bericht unter Verwendung von einem von Text oder Sprache erstellt wird.

8. Computerprogrammprodukt, das Anweisungen umfasst, die, wenn das Programm von einem Computer ausgeführt wird, den Computer veranlassen, die Schritte des Verfahrens nach einem der Ansprüche 1-4 auszuführen.

## Revendications

1. Procédé mis en œuvre par ordinateur pour identifier les examens médicaux antérieurs d'un patient, comprenant :
la réception d'une demande d'examens antérieurs pertinents pour un patient en provenance d'un dispositif de praticien utilisé par un professionnel de la santé, la demande incluant un examen actuel pour le patient, les examens antérieurs pertinents étant pertinents pour l'examen actuel ;
la détermination des examens antérieurs pertinents à partir d'examens antérieurs du patient sur la base d'un modèle personnalisé, le modèle personnalisé étant associé au professionnel de la santé, le modèle personnalisé indiquant un score de pertinence des examens antérieurs pertinents pour l'examen actuel, dans lequel le modèle personnalisé est basé sur un modèle de base et des données de rétroaction, dans lequel le modèle de base est créé sur la base des examens antérieurs et d'autres examens antérieurs pour d'autres patients ; et
la réception des examens antérieurs et d'autres examens antérieurs, le tri des examens antérieurs et des autres examens antérieurs ;
la génération d'une liste de paires d'examens antérieurs et d'autres examens antérieurs ; et
la détermination d'une étiquette de vérité terrain indiquant si chacune des paires est l'une d'une paire pertinente et d'une paire non pertinente ;
la transmission des examens antérieurs pertinents au dispositif de praticien ;
la réception d'un rapport en provenance du dispositif de praticien pour l'examen actuel, le rapport incluant une recommandation pour un autre acte à effectuer sur le patient ;
la détermination d'autres examens antérieurs pertinents à partir des examens antérieurs du patient sur la base du modèle de base ;
la détermination pour établir si les autres examens antérieurs pertinents rendent la recommandation inutile ; et
lorsque la recommandation est rendue inutile, la mise à jour du rapport pour supprimer la recommandation.

2. Procédé selon la revendication 1, dans lequel le modèle de base est en outre créé sur la base d'un extracteur de caractéristiques et d'un modèle statistique pour déterminer un score de pertinence de base pour chacune des paires qui est la paire pertinente.

3. Procédé selon la revendication 1, dans lequel les données de rétroaction incluent au moins une entrée reçue en provenance du dispositif de praticien pour une demande antérieure après que les examens antérieurs pertinents à la demande antérieure ont été reçus.

4. Procédé selon la revendication 1, dans lequel la recommandation est identifiée à l'aide d'une extraction de texte ou d'une détection vocale selon que le rapport est créé en utilisant du texte ou la parole.

5. Serveur d'optimisation comprenant :
un émetteur-récepteur communiquant par l'intermédiaire d'un réseau de communication, l'émetteur-récepteur recevant une demande d'examens antérieurs pertinents pour un patient en provenance d'un dispositif de praticien utilisé par un professionnel de la santé, la demande incluant un examen actuel pour le patient, les examens antérieurs pertinents étant pertinents pour l'examen actuel ; et
un processeur déterminant les examens antérieurs pertinents à partir d'examens antérieurs du patient sur la base d'un modèle personnalisé, le modèle personnalisé étant associé au professionnel de la santé, le modèle personnalisé indiquant un score de pertinence des examens antérieurs pertinents par rapport à l'examen actuel, dans lequel le modèle personnalisé est basé sur un modèle de base et des données de rétroaction, dans lequel le modèle de base est créé sur la base des examens antérieurs et d'autres examens antérieurs pour d'autres patients ; la réception des examens antérieurs et des autres examens antérieurs, le tri des examens antérieurs et des autres examens antérieurs, la génération d'une liste de paires des examens antérieurs et des autres examens antérieurs, et la détermination d'une étiquette de vérité terrain indiquant si chacune des paires est l'une d'une paire pertinente et d'une paire non pertinente ;
dans lequel l'émetteur-récepteur transmet les examens antérieurs pertinents au dispositif de praticien,
dans lequel l'émetteur-récepteur reçoit en outre un rapport en provenance du dispositif de praticien pour l'examen actuel, le rapport incluant une recommandation pour un autre acte à effectuer sur le patient, dans lequel le processeur détermine en outre d'autres examens antérieurs pertinents à partir des examens antérieurs du patient sur la base du modèle de base, détermine si les autres examens antérieurs pertinents rendent la recommandation inutile, et lorsque la recommandation est rendue inutile, met à jour le rapport pour supprimer la recommandation.

6. Serveur d'optimisation selon la revendication 5, dans lequel les données de rétroaction incluent au moins une entrée reçue en provenance du dispositif de praticien pour une demande antérieure après que les examens antérieurs pertinents à la demande antérieure ont été reçus.

7. Serveur d'optimisation selon la revendication 5, dans lequel la recommandation est identifiée à l'aide d'une extraction de texte ou d'une détection vocale selon que le rapport est créé en utilisant du texte ou la parole.

8. Produit de programme informatique comprenant des instructions qui, lorsque le programme est exécuté par un ordinateur, amènent l'ordinateur à réaliser les étapes du procédé selon l'une quelconque des revendications 1-4.
